# EUROPEAN PATENT APPLICATION

(11) **EP 1 295 593 A1**
(43) Date of publication of application: **26.03.2003**
(21) Application number: 02256577.4
(22) Date of filing: 23.09.2002
(51) Int. Cl.: A61K 7/48, A61K 7/50

(54) **Skin cleanser containing anti-aging active**

(30) Priority: 24.09.2001 US 961911
(71) Applicant: Johnson & Johnson Consumer Companies, Inc., Skillman, New Jersey 08558 (US)
(72) Inventor: Cole, Curtis A., Ringoes, NJ 08551 (US); Lukenbach, Elvin R., Flemington, NJ 0882 (US); Aleles, Margaret A., Gladstone, NJ 07934 (US)
(74) Representative: Mercer, Christopher Paul

(57) **Abstract**

The invention relates to a method of simultaneously cleansing the skin and providing an anti-aging skin benefit selected from the group consisting of skin firming, skin contouring, reducing the appearance of sagging skin, and skin tightening. The method comprises topically applying a skin cleanser composition comprising: (a) an effective amount of an anti-aging active compound of the formula: 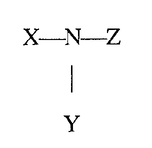 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group; (b) a cleansing surfactant; and (c) water. The skin cleanser compositions of the invention can be used as a 2-in-1 composition that simultaneously cleanses the skin and improves skin firmness and/or provides the skin with lifting benefits giving the user a fresh/alert appearance readily perceived by others.

## Description

### FIELD OF THE INVENTION

This invention relates to skin cleansers for improving skin contour by restoring youthful mechanical properties of the skin. More particularly, it relates to cleansing compositions containing at least one alkanolamine anti-aging active and their application to mammalian skin to increase the suppleness and compliance of affected skin areas. The skin cleanser compositions of the invention can be used as a 2-in-1 composition that simultaneously cleanses the skin and improves skin firmness and/or provides the skin with lifting benefits giving the user a fresh/alert appearance readily perceived by others.

### BACKGROUND OF THE INVENTION

Human beings have long sought products that can reverse or diminish the effects of aging without cosmetic surgery. The skin is composed primarily of water and as we age it loses its ability to retain water resulting in a surface that is dry and rough. This is not only due to a decrease in the water-retaining capacity of the stratum corneum, a decrease in barrier function and a decrease in the amount of secretion of sebum but also modulation of matrix molecules in the dermis that support epidermis. Matrix molecules comprised of proteins and polysaccharides become more crosslinked. This results in a decrease of water in the dermal compartment consequently producing a stiff, non-compliant structure. Aging of both skin and other tissues is, in part, the result of constant free radical damage leading to decreased cell function and matrix flexibility. In addition, sunlight exposure intensifies and augments the aging process. Extensive sun exposure results in photodamage and is manifested as lines, mottling, discoloration, precancers and cancers.

In addition to changes on the surface of the skin deeper changes occur in the dermis that effect it's mechanical properties making it more leathery and hardened. Free radical damage induces abnormal interfibrillar crosslink formation. This leads to degradation and atrophy of the matrix. As these crosslinks are formed dermal water is excluded.

Current treatments for these environmental insults include moisturizers, chemical peels, and laser surgery to either hydrate the surface of the skin, enhance epidermal turnover or remove the "hardened" dermal or supportive layers. The more aggressive procedures remove skin tissue and enhance epidermal regeneration and new matrix synthesis thereby restoring some of the original mechanical properties associated with young, undamaged skin.

Japanese Kokai Patent Application No. 495008 discloses an aging inhibitor effect of ethanolamine derivatives which indicates a preventative activity of aging skin effects. According to this application, the age inhibiting compound can be used as a therapeutic to treat aged skin to improve skin elasticity and wrinkles.

U.S. Patent No. 5,554,647 to Perricone discloses a method for percutaneously treating aging skin using ethanolamine ingredients in a dermatologically acceptable carrier. The ethanolamine is selected from the group consisting of dimethylaminoethanol, monoaminoethanol, choline, serine, acetic acid esters of dimethylaminoethanol, acetic acid esters of monoaminoethanol, para-chlorophenylacetic acid esters of dimethylaminoethanol, para-chlorophenylacetic acid esters of monoaminoethanol, and mixtures thereof. These compounds are hypothesized to treat aging skin via a mechanism of neuromuscular stimulation.

Not wishing to be bound to this hypothesis, we have additionally discovered that the superficial biomechanical properties of the skin are affected by application of these same compounds restoring youthful firmness resulting in improved facial contours. Further, U.S. Patent No. 5,554,647 does not disclose cleansing compositions.

Surfactant cleansers are typically unable to deliver water soluble "actives" as they are washed away from the face or body with the cleanser foam and are not able to effectively penetrate in typical use timing. Some surfactant systems are used to deliver oil soluble "actives" such as acne ingredients or moisturizers, but tend to have low foaming capabilities and are not well noted for their cleansing properties.

C-ESTA™ distributed by Jan Marini contains a DAE-complex (a complex containing dimethaminoethanol and a vitamin C palmitate) wherein dimethaminoethanol is disclosed as a penetration enhancer, enabling the vitamin C palmitate to penetrate the skin for antioxidant benefits.

It is an object of this invention to provide dual purpose cleanser systems that are capable of delivering anti-aging benefits (e.g., skin firming, skin contouring, reducing the appearance of sagging skin, and skin tightening) at the same time as cleansing. This would enable a 2-in-1 system that would allow consumers to appreciate anti-aging benefits without having to alter their routine skin cleansing process or apply a second treatment product. The cleansing compositions of the invention can be used to improve facial contours by modulating the skin's biomechanical properties producing more youthful characteristics.

### SUMMARY OF INVENTION

Accordingly, the invention relates to a method of simultaneously cleansing the skin and providing an anti-aging skin benefit selected from the group consisting of skin firming, skin contouring, reducing the appearance of sagging skin, and skin tightening. The method comprises topically applying a skin cleanser composition comprising:
(a) an effective amount of an anti-aging active compound of the formula: 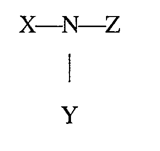 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group;
(b) a cleansing surfactant; and
(c) water.

In another embodiment, the invention relates to a skin cleanser composition comprising:
(a) an effective amount of an anti-aging active compound of the formula: 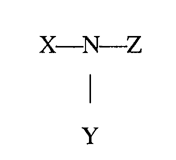 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group;
(b) a cleansing surfactant; and
(c) water;
wherein said composition is substantially free of vitamin C and derivatives thereof.

In yet another embodiment, the invention relates to a skin cleanser composition comprising:
(a) an effective amount of an anti-aging active compound of the formula: 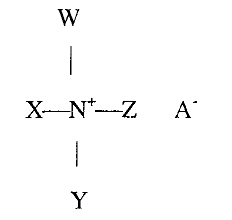 wherein W, X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group, and wherein A is a mixture of anionic counterions derived from at least two pharmaceutically acceptable acids and esters thereof;
(b) a cleansing surfactant; and
(c) water.

### BRIEF DESCRIPTION OF THE DRAWINGS

Figure 1 is a bar graph showing the results of the grader evaluation of the cleansing compositions of Examples 1-4 when compared to placebo as described in Example 5.
Figure 2 is a bar graph showing the results of the panelist evaluation of the cleansing compositions of Examples 1-4 when compared to placebo as described in Example 5.

### DETAILED DESCRIPTION OF THE INVENTION

The alkanolamine anti-aging active used in the methods according to the invention has the following general formula: 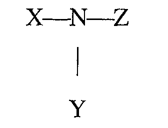 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group.

In a preferred embodiment the alkanolamine is selected from the group consisting of ethylaminoethanol, methylaminoethanol, dimethylaminoethanol, isopropanolamine, triethanolamine, isopropanoldimethylamine, ethylethanolamine, 2-butanolamine, choline and serine. More preferably, the alkanolamine is dimethylaminoethanol (DMAE).

The cleansing compositions used in the methods according to the invention preferably contain from about 0.1 about 10% by weight of the alkanolamine, more preferably, from about 0.1 to about 5% and, most preferably, from about 1 to about 3% by weight based on the total composition.

In a preferred embodiment, the compositions used in the methods of the invention contain a pH buffering agent. Preferably, the amount of buffering agent should be that which would result in compositions having a pH ranging from about 4.0 to about 9.0, more preferably from about 5.5 to about 7.0. The buffering agent can be any of the known buffering agents commonly found in cosmetic compositions provided that they are physically and chemically stable with the other ingredients of the composition. Suitable buffering agents include but are not intended to be restricted to organic acids such as citric acid, malic acid, and glycolic acid.

Another compound which is advantageously present in the compositions of this invention is tyrosine. Tyrosine may be present in the compositions of this invention in the amount of from about 0.01 to about 5%, more preferably from about 0.04 to about 3% by weight and most preferably about 0.5% by weight, based on the total composition.

The cleansing compositions according to the invention can comprise additional ingredients commonly found in skin care compositions, such as for example, emollients, skin conditioning agents, emulsifying agents, humectants, preservatives, antioxidants, perfumes, chelating agents, etc., provided that they are physically and chemically compatible with the other components of the composition. Notably useful is the incorporation of vitamin A and vitamin A derivatives, including but not restricted to retinol, retinyl palmitate, retinoic acid, retinal, and retinyl propionate.

Examples of suitable preservatives for use in the compositions of the invention include the C₁-C₄ alkyl parabens and phenoxyethanol. Generally, the preservative is present in an amount ranging from about 0.5 to about 2.0, preferably about 1.0 to about 1.5, weight percent based on the total composition. In a preferred embodiment, the preservative is mixture of from about 0.2 to about 0.5 weight percent methylparaben, from about 0.2 to about 5.0 weight percent propylparaben and from about 0.05 to about 0.10 weight percent butylparaben. A particularly preferred commercially available preservative that may be used in the skin care composition according to this invention is PHENONIP TM which is a practically colorless, viscous, liquid mixture of phenoxyethanol, methylparaben, ethylparaben, propylparaben, and butylparaben available from Nipa Laboratories, Inc., Wilmington, Del.

Preferably, antioxidants should be present in the compositions according to the invention. Suitable antioxidants include butylated hydroxy toluene (BHT), ascorbyl palmitate, butylated hydroxyanisole (BHA), phenyl-α-naphthylamine, hydroquinone, propyl gallate, nordihydroquiaretic acid, vitamin E or derivatives of vitamin E, vitamin C or derivatives of vitgamin C, calcium pantothenic, green tea extracts and mixed polyphenosls, and mixtures thereof of the above. When utilized the antioxidant can be present in an amount ranging from about 0.02 to about 0.5% by weight, more preferably from about 0.002 to about 0.1% by weight of the total composition.

Emollients which can be included in the compositions of the invention function by their ability to remain on the skin surface or in the stratum corneum to act as lubricants, to reduce flaking, and to improve the skin appearance. Typical emollients include fatty esters, fatty alcohols, mineral oil, polyether siloxane copolymers and the like. Examples of suitable emollients include, but are not limited to, polypropylene glycol ("PPG")-15 stearyl ether, PPG-10 cetyl ether, steareth-10, oleth-8, PPG-4 lauryl ether, vitamin E acetate, PEG-7 glyceryl cocoate, lanolin, cetyl alcohol, octyl hydroxystearate, dimethicone, and combinations thereof. Cetyl alcohol, octyl hydroxystearate, dimethicone, and combinations thereof are preferred. When utilized, the emollient can be present in an amount from about 0.01 to about 5, preferably from about 1 to about 4 percent by weight based on the total composition.

Polyhydric alcohols can be utilized as humectants in the compositions of the invention. The humectants aid in increasing the effectiveness of the emollient, reduce scaling, stimulate removal of built-up scale and improve skin feel. Suitable polyhydric alcohols include, but are not limited to, glycerol (also known as glycerin), polyalkylene glycols, alkylene polyols and their derivatives, including butylene glycol, propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin is preferred. When utilized, the humectant is present in an amount from about 0.1 to about 10, preferably from about 1 to about 7 percent by weight, based on the total weight of the composition.

The compositions according to the invention preferably contain an effective stabilizing amount of a stabilizing emulsifier in stabilizing the composition from phase separation. Preferably, the stabilizing emulsifier is present at from about 1.0 to about 10.0, more preferably from about 3.0 to about 6.0, weight percent, based on the total composition. Any emulsifier that is compatible with the components of the composition can be employed. Suitable stabilizing emulsifiers include stearic acid, cetyl alcohol, stearyl alcohol, steareth 2, steareth 20, Acrylates/C10-30 alkyl Acrylate Crosspolymer. Particularly preferred is PEMULEN TR-1 (CTFA Designation: Acrylates/10-30 Alkyl Acrylate Crosspolymer).

Any fragrance may be added to the compositions of the invention for aesthetic purposes. Suitable fragrances include, but are not limited to, eucalyptus oil, camphor synthetic, peppermint oil, clove oil, lavender, chamomile and the like. When utilized, fragrances are present in an amount from about 0.05 to about 0.5, preferably from about 0.1 to about 0.3 percent by weight, based on the total weight of the composition.

In certain aspects of this invention, the compositions should include a chelating agent. Chelating agents which are useful in the compositions of the present invention include ethylenediamine tetra acetic acid (EDTA) and derivatives and salts thereof, dihydroxyethyl glycine, tartaric acid, and mixtures thereof. The chelating agents should be utilized in a stabilizing effective amount and may range from about 0.01 to about 2% based on the weight of the total composition, preferably from about 0.05 to about 1%. Most preferably, the chelating agent should be EDTA.

In one embodiment, the cleansing composition of the invention is substantially free of vitamin C and derivatives thereof such as ascorbyl palmitate, sodium ascorbyl phosphate, magnesium ascorbyl phosphate and similar type molecules. By "substantially free" it is meant less than 0.5% by weight of the cleansing composition. In another embodiment, the cleansing composition is free from vitamin C and derivatives thereof.

The skin cleanser may be in the form of an oil-in-water emulsion, a water-in-oil emulsion, dispersion, microemulsion, or a lamellar (liquid crystalline) composition. The skin cleanser of the present invention comprises at least one cleansing surfactant including foaming surfactants and non-foaming surfactants. Suitable surfactants include non-ionic, cationic, amphoteric, or anionic surfactants; nonionic surfactants are preferred. By "foaming," it is meant that the surfactant, when used with the composition of the present invention, has a column height of foam greater than about 20 mm as determined by the Ross-Miles Foam Generation Test. See 18 (I.) Oil & Soap 99 - 102 (1941) ("Ross-Miles Test"), which is incorporated by reference herein. As used herein, the term "amphoteric" shall mean: 1) molecules that contain both acidic and basic sites such as, for example, an amino acid containing both amino (basic) and acid (e.g., carboxylic acid, acidic) functional groups; or 2) zwitterionic molecules which possess both positive and negative charges within the same molecule. The charges of the latter may be either dependent on or independent of the pH of the composition. Examples of zwitterionic materials include, but are not limited to, alkyl betaines and amidoalkyl betaines. Examples of suitable and preferred foaming and non-foaming surfactants may be found in copending application Serial No. 09/604,563, filed June 27, 2000, which is incorporated by reference in its entirety herein. Specific examples of suitable foaming surfactants include sodium cocoyl sarcosinate, decyl glucoside, lauryl glucoside, ammonium laureth sulfate, cocoamidopropyl betaine, lauryl betaine, sodium cocoamphoacetate, and mixtures thereof. Generally, the foaming surfactant should be present in an amount to provide effective cleansing properties. In one embodiment, the foaming surfactant is present in an amount ranging from about 10 to about 40% by weight of the cleansing composition. For purposes of economy and for low intensity of action to reduce drying and irritancy for facial use, in one embodiment, the foaming surfactant is present at from about 5 to about 20% by weight of the cleansing composition.

Examples of suitable non-foaming surfactants include non-foaming nonionic surfactants such as sucrose esters, e.g., sucrose cocoate, sucrose stearate and mixtures thereof, with sucrose cocoate being preferred. By "non-foaming," it is meant that the surfactant, when used with the composition of the present invention, has a column height of less than about 20 mm as determined by the Ross-Miles Test. A preferred combination of non-foaming surfactant and hydrophilic components include, based upon the total weight percent of the skin cleanser, from about 0.1 percent to about 5.0 percent of hexylene glycol, from about 0.5 percent to about 3.0 percent of sucrose cocoate non foaming surfactant, and from about 0.5 percent to about 3.0 percent of polyoxyethylene-6 caprylic/capric triglyceride. An example of a suitable cleaning enhancer include a mixture of sorbitan stearate and sucrose cocoate available from Uniqema under the tradename, "Arlatone 2121." Preferably, the skin cleanser contains, based upon the total weight of the skin cleanser, no more than about 6%, and preferably 5%, of the non-foaming surfactant.

The skin cleanser of the present invention may also optionally contain a cleaning enhancer in the form of a nonionic emulsifier. Examples of suitable nonionic emulsifiers include isocetheth-20, oleth-2, mixture of PEG-40 hydrogenated castor oil and trideceth-9 available from Dragoco Inc. under the tradename, "Dragoco Solubilizer 2/014160," Poloxamer 184, laureth-4, sorbitan trioleate, polyoxyethylene-(2) oleyl ether, PEG-20 methyl glucose sesquistereate, methyl glucose dioleate, sorbitan stearate, cetearyl glucoside, glyceryl oleate, trideceth-9, polyethylene glycol-40 hydrogenated castor oil, and mixtures thereof.

Preferably, the skin cleanser contains, based upon the total weight of the skin cleanser, no more than about 6%, and preferably 5%, of the cleaning enhancers for cream formulations and no more than about 2%, and preferably no more than 1% of the cleaning enhancers in thin lotion/milk formulations.

The above described skin cleanser may be prepared by combining the desired components in a suitable container and mixing them under ambient conditions in any conventional mixing means well known in the art, such as a mechanically stirred propeller, paddle, and the like.

Generally, the cleansing composition is topically applied to the affected skin areas in a predetermined or as-needed regimen to bring about improvement, it generally being the case that gradual improvement is noted with each successive application. Insofar as has been determined based upon clinical studies to date, no adverse side effects are encountered. The skin cleanser composition according to the invention may be in the form of a gel, a bath, a wash, a mousse, a shampoo, a rinse, a lotion, a cream, or a spray. The compositions of the present invention may be directed applied to the skin or may be applied onto other delivery implements such as wipes, sponges, brushes, puffs and the like. The compositions may be used in products designed to be left on the skin, wiped from the skin, or rinsed off of the skin.

The skin cleansing compositions according to the invention can be used as a facial cleanser or as a full body cleanser. For example, the skin cleanser composition can be applied to the face, legs, thighs, arms, breasts or any other area of the skin in which anti-aging benefits are desired. Surprisingly, the cleanser compositions of the invention may be used in products designed to be rinsed off of the skin even though the anti-aging active (alkanolamine) is water soluble. Indeed, it has been discovered that the anti-aging active can be delivered in a surfactant system to provide anti-aging benefits after contact with the skin for as little as one minute and then rinsed with water.

In yet another embodiment, the invention relates to a skin cleansing composition comprising an effective amount of an anti-aging active compound of the formula: 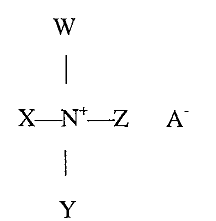 wherein W, X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group, and wherein A is a mixture of anionic counterions derived from at least two pharmaceutically acceptable acids and esters thereof; a cleansing surfactant; and water.

The alkanoamine salt can be made by techniques known in the art by reacting the desired alkanolamine with an appropriate acid under conditions sufficient to form the salt. The salts can be formed *in situ* or prior to formulating. Generally, when at least one of W, X, Y, or Z is hydrogen the alkanolamine salt can be prepared *in situ.*

In a preferred embodiment, the alkanoloamine salt is an acid salt of monomethylaminoethanol, dimethylaminoethanol, trimethylammonioethanol hydroxide, isopropanolamine, triethanolamine, isoropanoldimethylamine, ethylethanolamine, 2-butanolamine, choline, serine, and copolymers thereof.

Suitable acids for use in the preparation of the alkanolamine salts according to the invention include any organic acid known to be useful in skin care compositions. In a preferred embodiment, at least one of the acids is an alpha hydroxy acid, such as taught for example in U.S. Patent No. 5,856,357, the disclosure of which is hereby incorporated by reference. Particularly preferred is a mixture of at least two of glycolic acid, malic acid and citric acids. In a most preferred embodiment, the acid is a combination of glycolic acid and either malic or citric acid. In situations where pH stability is a particular concern, e.g., long term storage, a particularly preferred embodiment is when the acid is a mixture of citric and glycolic acid. Preferably, the ratio of malic or citric acid to glycolic acid ranges from about 1:1 to about 1:5, more preferably, from about 1:1 to about 1:3.

The advantages of the invention and specific embodiments of the skin care compositions prepared in accordance with the present invention are illustrated by the following examples. It will be understood, however, that the invention is not confined to the specific limitations set forth in the individual examples, but rather defined within the scope of the appended claims.

### EXAMPLE 1

The following formula was made in accordance with the teachings of this invention. The cleanser was prepared separately and added to a suitable closed container with slow mixing to avoid foaming. The DMAE/water premix was added to the container and mixed well. The citric and glycolic acids were added to water then added to cleanser and DMAE mix. The specific ingredients and weight percentages thereof are tabulated below.

| **INGREDIENT:** | **WEIGHT PERCENT:** |
|---|---|
| ***Cleanser:*** | |
| Deionized water | 41.50 |
| Tetrasodium EDTA | 0.08 |
| Glycerine | 5.97 |
| Glycereth-7 | 1.70 |
| Sodium cocoyl sarcosinate | 4.26 |
| Decyl glucoside | 6.39 |
| Ammonium laureth sulfate | 6.39 |
| Cocoamide DEA | 1.70 |
| Cocoamidopropyl betaine | 6.39 |
| Lauryl glucoside | 8.52 |
| PEG-120 Methyl glucose dioleate | 0.51 |
| Glycol stearate | 0.85 |
| Citric acid (20% sol.) | 0.25 |
| DMDM Hydantoin | 0.17 |
| Fragrance | 0.51 |

| ***DMAE Premix:*** | |
|---|---|
| Deionized water | 6.0 |
| DMAE | 3.0 |

| ***Buffer Premix:*** | |
|---|---|
| Glycolic acid (70%)/water (30%) | 3.3 |

### EXAMPLE 2

The following formula was made in accordance with the teachings of this invention. Deionized water tetrasodium EDTA, glycerine and glycereth-7 were mixed in the main kettle and heated to about 60°C. Sodium cocoyl sarcosinate was added and heating stopped. Decyl polyglucose, ammonium laureth sulfate, PPG-3 hydroxyethyl linoleamide, cocamidopropyl betaine, decyl glucoside (90% reserve 10% for fragrance addition) were added and the mixture was stirred. Then PEG-120 methyl glucose dioleate was added and the composition was cooled to about 30°C. At 30°C DMDM Hydantoin, mixture of glycol distearate and glycerin and lauryth-4 and cocodimidopropyl betaine available from Henkel under the name Euperlan PK 3000 OK and fragrance were added. The pre-mix of DMAE, water, citric acid and glycolic acid was then added. The pH of the product was adjusted to pH 5.5 with glycolic acid (70%)/ water (30%) solution. The specific ingredients and weight percentages thereof are tabulated below.

| **INGREDIENT:** | **WEIGHT PERCENT:** |
|---|---|
| ***Cleanser:*** | |
| Deionized water | 39.70 |
| EDTA disodium | 0.10 |
| Glycerine | 7.00 |
| Glycereth-7 | 2.00 |
| Sodium cocoyl sarcosinate | 5.00 |
| Decyl glucoside | 7.50 |
| Ammonium laureth sulfate | 7.50 |
| PPG-3 Hydroxyethyl linoleamide | 2.00 |
| Cocoamidopropyl betaine | 7.50 |
| Lauryl glucoside | 10.00 |
| PEG-120 Methyl glucose dioleate | 1.50 |
| Mixture of glycol distearate and glycerin and lauryth-4 and cocodimidopropyl betaine (Euperlan PK 3000 OK) | 2.80 |
| Glycolic acid (70% sol.) | 3.30 |
| DMDM Hydantoin | 1.00 |
| Fragrance | 0.40 |

| ***DMAE Premix:*** | |
|---|---|
| Deionized water | 3.0 |
| DMAE | 3.0 |

| ***Buffer Premix:*** | |
|---|---|
| Glycolic acid (70%)/water (30%) | 3.3 |

### Example 3

The following formula was made in accordance with the teachings of this invention. Deionized water was heated to about 85°C and then STABILEZE QM a DVM/MA decadiene crosspolymer available from ISP was added. Once the STABILEZE QM completely dissolved the temperature of the mixture was reduced to about 65°C. Sodium laureth sulfate was then added followed by sodium cocoamphoacetate. A premix of glycolic acid and DMAE was then added. When the temperature of the mixture reached about 60°C, methyl paraben and capryoyl glycine were added. The mixture was then cooled. At about 50°C laurylglucoside and laurylbetaine (5 ml reserved for fragrance addition) were added. At about 35°C, glycerine, sodium citrate, EUPERLAN and EDTA disodium were added. The remaining 5 ml of laurylbetaine was mixed with the fragrance and then added to the batch when the temperature reached about 30°C. The pH of the product was adjusted to pH 5.5 with 20% potassium hydroxide solution. The specific ingredients and weight percentages thereof are tabulated below.

| **INGREDIENT:** | **WEIGHT PERCENT:** |
|---|---|
| ***Cleanser:*** | |
| Deionized water | 77.00 |
| Sodium cocoamphoacetate | 2.50 |
| Sodium Laureth Sulfate | 3.00 |
| Methyl Paraben | 4.00 |
| DVM/MA decadiene crosspolymer (Stabileze QM) | 1.20 |
| Lauryl glucoside | 3.8 |
| Laurylbetaine | 3.0 |
| Capryloyl Glycine | 0.70 |
| Glycerine | 4.00 |
| Disodium EDTA | 0.10 |
| Sodium Citrate | 0.30 |
| Euperlan K 3000 | 2.00 |
| Glycolic acid (70% sol.) | 3.50 |
| Fragrance | 0.40 |

| ***DMAE Premix:*** | |
|---|---|
| Deionized water | 3.0 |
| DMAE | 3.0 |

| ***Buffer Premix:*** | |
|---|---|
| Glycolic acid (70%)/water (30%) | 3.59 |

### EXAMPLE 4

The following formula was made in accordance with the teachings of this invention. Deionized water was heated to about 85°C and then STABILEZE QM a DVM/MA decadiene crosspolymer available from ISP was added. Once the STABILEZE QM completely dissolved the temperature of the mixture was reduced to about 60°C. Tetrasodium EDTA, glycerine, and glycereth-7 were added. DMAE and glycolic acid 70%/ water 30% solution were mixed and then added to the batch. The batch was mixed for 15 minutes until dispersed and then heated to about 60°C . Sodium cocoyl sarcosinate was then added and heating stopped. Decyl glucoside, ammonium laureth sulfate, cocoamide DEA, cocamidopropyl betaine and lauryl glucoside (60 ml reserved for fragrance addition) was then added and mixed for 20 minutes or until no gel or lumps remained. Then the mixture was heated to about 65°C and glycol stearate was added. The mixture was then mixed for 30 minutes or until no white flakes remained. At about 40°C DMDM hydantoin was added and the remaining 60 ml of lauryl glucoside premixed with the fragrance was added to the batch. The pH of the product was adjusted to pH 5.5 with glycolic acid (70%)/ water (30%) solution. The specific ingredients and weight percentages thereof are tabulated below.

| **INGREDIENT:** | **WEIGHT PERCENT:** |
|---|---|
| ***Cleanser:*** | |
| Deionized water | 41.00 |
| Tetrasodium EDTA | 0.10 |
| Glycerine | 7.00 |
| Glycereth-7 | 2.00 |
| Sodium cocoyl sarcosinate | 5.00 |
| Decyl glucoside | 7.50 |
| Ammonium laureth sulfate | 7.50 |
| Cocamide DEA | 2.00 |
| Cocoamidopropyl betaine | 7.50 |
| Lauryl glucoside | 10.00 |
| DVM/MA decadiene crosspolymer (Stabileze QM) | 1.20 |
| Glycol stearate | 1.00 |
| Glycolic acid (70% sol.) | 3.00 |
| DMDM Hydantoin | 0.20 |
| Fragrance | 0.60 |

| ***DMAE Premix:*** | |
|---|---|
| Deionized water | 3.0 |
| DMAE | 3.0 |

| ***Buffer Premix:*** | |
|---|---|
| Glycolic acid (70%)/water (30%) | 3.0 |

### EXAMPLE 5

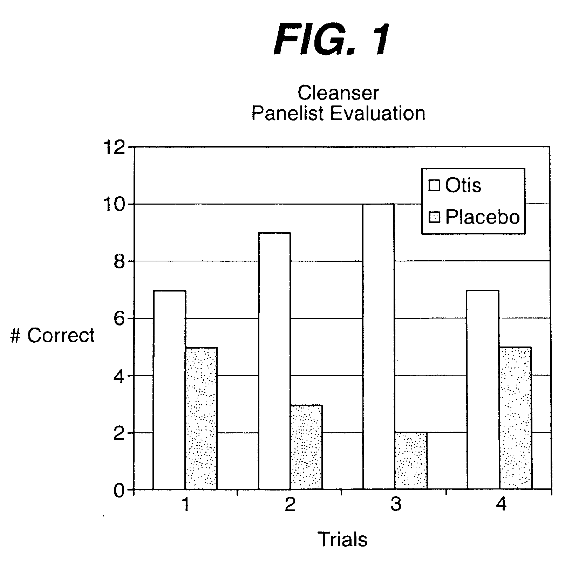

Examples 1 - 4 were evaluated on panels of 12 panelists. The panelists were instructed to wet their face, apply the composition on one side of the face, rubbing to lather the product on that side of the face only, and allow the lather to stay on the face for one minute, then rinse completely with water. The corresponding placebo cleanser, not containing the DMAE, was applied similarly to the opposite side of the face (right or left side of face) with the same instructions. Sites of application of the two formulae was randomly distributed across the panel of 12 subjects. One hour after application, the panelists were observed by a grader (blinded as to the product assignments) who made assessment as to which side of the face was treated with the active DMAE containing product based on the skin lifting, skin contouring and skin firming effects of the DMAE on the facial features. The grader was correct in identifying the active treated for 11 of the 12 subjects (p<0.05) for Example 1, concluding that the cleanser system was effective in delivering the lifting/firming benefits, even though the active is highly water soluble. Similar results were shown for Examples 2-4. The results of the grader evaluations for each of Examples 1-4 are described in Figure 1, showing significant correlation of lifting and firming benefits with the use of the inventive examples when compared to the placebos.

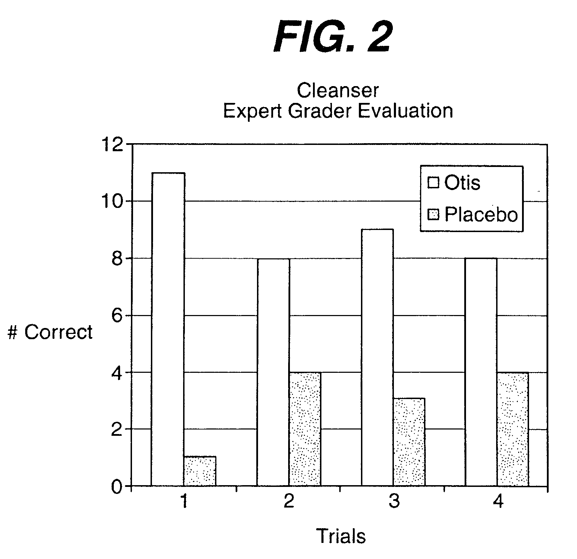

Each of the panelists were also asked to make assessment as to which side of the face was treated with the active DMAE containing product based on the skin lifting, skin contouring and skin firming effects of the DMAE on the facial features. Some panelists noted skin firming and lifting benefits as early as 5 minutes after rinsing product after only one minute of skin contact of the cleanser during the cleansing process. The results of the panelist evaluations for each of Examples 1-4 are described in Figure 2, showing significant correlation of lifting and firming benefits with the use of the inventive examples when compared to the placebos.

### EXAMPLE 6

The following emulsion was made in accordance with the teachings of this invention and then impregnated onto a nonwoven substrate to form a wet cleansing wipe.
A. Preparation of Oil Phase: Isononyl isonnaoate, cyclomethicone, Isostearyl Palmitate, Cetyl Octanoate, Pentaerythritol Tetraoctanoate, PEG-20 Methyl Glucose Sesquistearate and Methyl Glucose Dioleate were mixed together and heated to 45°C. Acrylates C10-C30 alkyl acrylate crosspolymer (available from B.F. Goodrich under the name PEMULEN TR-1) was then added with mixing until all particles dispersed.
B. Preparation of Water Phase: In a separate beaker sucrose cocoate, PEG-6 Capric/Caprylic Glycerides, hexylene glycol, methylparaben, propylparaben were mixed together and then added to water and heated to 45°C.

The oil phase was added to the water phase with medium stirring and mixed until uniform. The mixture was allowed to cool. When the temperature was about 40°C, the DMAE premix was added. The pH of the emulsion was adjusted to about 7.0 using the buffer premix of water, glycolic acid and malic acid.

| **INGREDIENT:** | **WEIGHT PERCENT:** |
|---|---|
| ***Oil Phase:*** | |
| Isononyl Isononaoate | 1.50 |
| Cyclomethicone | 1.50 |
| Isostearyl Palmitate | 1.50 |
| Cetyl Octanoate | 1.50 |
| Pentaerythritol Tetraoctanoate | 1.50 |
| PEG-20 Methyl Glucose Sesquistearate | 1.30 |
| Methyl Glucose Dioleate | 0.70 |
| Acrylates C10-C30 alkyl acrylate crosspolymer (PEMULEN TR-1) | 0.50 |

| ***Water Phase*** | |
|---|---|
| Deionized water | 65.18 |
| PEG-6 Capric/Caprylic Glycerides | 0.75 |
| Hexylene Glycol | 1.00 |
| Sucrose Cocoate | 0.60 |
| Methylparaben | 0.30 |
| Propylparaben | 0.05 |

| ***Post Additions*** | |
|---|---|
| ***DMAE Premix:*** | |
| DMAE | 3.00 |
| Deionized water | 15.00 |
| Fragrance | 0.20 |

| ***Buffer Premix*** | |
|---|---|
| Deionized water | 1.54 |
| Glycolic acid (70%) | 1.40 |
| Malic acid | 0.98 |

Having described the invention with reference to particular compositions, theories of effectiveness, and the like, it will be apparent to those of skill in the art that it is not intended that the invention be limited by such illustrative embodiments or mechanisms, and that modifications can be made without departing from the scope or spirit of the invention, as defined by the appended claims. The claims are meant to cover the claimed components and steps in any sequence which is effective to meet the objectives there intended, unless the context specifically indicates the contrary.

## Claims

1. A skin cleanser composition comprising:
(a) an effective amount of an anti-aging active compound of the formula: 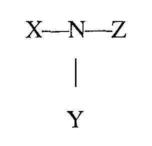 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group;
(b) a cleansing surfactant; and
(c) water,
for use in a method of simultaneously cleansing the skin and providing an anti-aging skin benefit selected from the group consisting of skin firming, skin contouring, reducing the appearance of sagging skin, and skin tightening by topical application of said composition.

2. The composition for use of claim 1, wherein said anti-aging active compound is ethylaminoethanol, methylaminoethanol, dimethylaminoethanol-amine, isopropanolamine, triethanolamine, isopropanoldimethylamine, ethylethanolamine, 2-butanolamine, choline or serine.

3. The composition for use of claim 2, wherein said anti-aging active is dimethylaminoethanol.

4. The composition for use of any one of claims 1 to 3, wherein said anti-aging active is present in an amount of from 0.1 to 10% by weight of the composition.

5. The composition for use of any one of claims 1 to 4, wherein the skin cleanser composition further comprises tyrosine.

6. The composition for use of any one of claims 1 to 5, wherein said skin cleansing composition is for application to at least one of the face, legs, thighs, arms, chests and breasts.

7. The compositon for use of any one of claims 1 to 6, wherein the skin cleanser composition is in the form of a gel, a bath, a wash, a mousse, a shampoo, a rinse, a lotion, a cream, or a spray.

8. The composition for use of any one of claims 1 to 7, wherein the skin cleanser composition is incorporated into material carrier selected from a wet wipe, a puff, a brush, or a sponge.

9. A skin cleanser composition comprising:
(a) an effective amount of an anti-aging active compound of the formula: 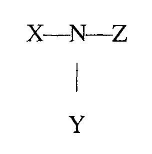 wherein X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group;
(b) a cleansing surfactant; and
(c) water
wherein said composition is substantially free of vitamin C and derivatives thereof.

10. A skin cleanser composition comprising:
(a) an effective amount of an anti-aging active compound of the formula: 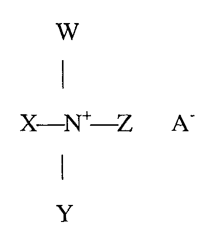 wherein W, X, Y and Z are selected from the group consisting of hydrogen, C₁-C₃ alkyl group, C₂-C₄ alkanol group, wherein at least one of X, Y or Z is a C₂-C₄ alkanol group bearing at least one hydroxyl group and optionally at least one carboxyl group, and
wherein A is a mixture of anionic counterions derived from at least two pharmaceutically acceptable acids and esters thereof;
(b) a cleansing surfactant; and
(c) water.
